# EUROPEAN PATENT APPLICATION

(11) **EP 1 178 029 A1**
(43) Date of publication of application: **06.02.2002**
(21) Application number: 00116504.2
(22) Date of filing: 31.07.2000
(51) Int. Cl.: C07C 9/21, C07C 11/02, C07C 31/125, C07C 53/126, C10L 1/16

(54) **Process for preparing di-iso-butanes, di-iso-butenes, and di-n-butenes from field butanes**

(71) Applicant: Oxeno Olefinchemie GmbH, 45764 Marl (DE)
(72) Inventor: Nierlich, Franz, Dr., 45768 Marl (DE); Olbrich, Paul, Dr., 45721 Haltern (DE); Droste, Wilhelm, Dr., 45770 Marl (DE); Müller, Richard, Dr., 45770 Marl (DE); Tötsch, Walter, Dr., 45770 Marl (DE)

(57) **Abstract**

The invention relates to a process for preparing butene oligomers and di-iso-butanes from field butanes, in which (a) the n-butane and iso-butane present in the field butanes **1** are dehydrogenated in a dehydrogenation stage **2,** (b) the dehydrogenation mixture **3** is oligomerized in an oligomerization stage **8,** (c) the oligomerisation mixture is separated in di-iso-butenes, di-n-butenes, and residual gas, and (d) hydrogenating the di-iso-butenes to give di-iso-butanes. The di-iso-butanes can be used as fuel additive. The oligomerization products di-n-butene and tri-butene are starting materials for preparing plasticizer alcohols and detergent bases. By incorporating an isomerization stage **24**, the process may be controlled in such a manner that the only di-butene formed is di-n-butene or di-iso-butene.

## Description

The invention relates to a process for preparing butene oligomers, which are valuable starting materials for plasticizer alcohols, from field butanes. Preferred butene oligomers are the isomeric octenes which are dimeric butenes and are therefore also termed di-butene. Di-butenes particularly in demand are di-n-butene and di-iso-butene. The invention therefore also relates to a process in which di-iso-butene is separated off from di-butene and further processed.

Di-butene is an isomeric mixture which is formed, in addition to higher butene oligomers, by dimerization and/or codimerization of butenes, i.e. of n-butene and/or isobutene, in the oligomerization of butenes. The term di-n-butene is applied to the dimerization product of n-butene, i.e. of 1-butene and/or of 2-butene. Important components of di-n-butene are 3-methyl-2-heptene, 3,4-dimethyl-2-hexene and, to a lesser extent n-octenes. Di-isobutene is the mixture of dimers which is formed by dimerization of isobutene. Di-isobutene contains molecules which are more highly branched than di-butene, and this in turn is more highly branched than di-n-butene.

Di-butene, di-n-butene and di-iso-butene are starting materials for preparing isomeric nonanols by hydroformylation and hydrogenation of the C₉ aldehydes thus formed. Esters of these nonanols, in particular the phthalic esters, are plasticizers, which are prepared to a significant extent, and are primarily used for poly(vinyl chloride). Nonanols from di-n-butene are linear to a greater extent than nonanols from di-butene, which are in turn less branched than nonanols from di-isobutene. Esters of nonanols from di-n-butene, because of their more linear structure, have application advantages in comparison with esters from nonanols based on di-butene and di-isobutene and are particularly in demand.

Butenes can be obtained for the dimerization from the C₄ fraction of steam crackers or of FC crackers, for example. This fraction is generally worked up, by first separating off 1,3-butadiene by a selective scrubbing, e.g. using n-methylpyrrolidone. Isobutene is a desirable and particularly valuable C₄ fraction component, because it may be chemically reacted to give sought-after products, e.g. with iso-butane to give high-octane isooctane or with methanol to give methyl tert-butyl ether (MTBE), which, as an additive to motor gasoline, improves its octane rating. After the reaction of the isobutene, the n-butenes and n-butane and iso-butane remain behind. The proportion of n-butenes in the cracking products of the steam cracker or the FC cracker is relatively low, however, that is in the order of magnitude of barely 10 per cent by weight, based on the principal target product ethylene. A steam cracker having the respectable capacity of 600,000 metric t/year of ethylene therefore only delivers around 60,000 metric t/year of n-butene. Although its amount (and that of the isobutenes) could be increased by dehydrogenating the around 15,000 metric t/year of n-butane and iso-butane which arise in addition to the n-butenes, this is not advisable, because dehydrogenation plants require high capital expenditure and are uneconomic for such a small capacity.

Isobutene is, as stated, a cracking product in demand, and is therefore not generally available for the oligomerization. The amount of n-butenes which a steam cracker or an FC cracker produces directly is not sufficient, however, to produce sufficient di-butene for a nonanol plant whose capacity is so high that it could compete economically with the existing large-scale plants for preparing important plasticizer alcohols, such as 2-ethylhexanol. N-butenes from various steam crackers or FC crackers would therefore have to be collected and oligomerized together, in order to cover the di-butene demand of a large nonanol plant. Opposing this, however, is the fact that the transport of liquified gases is expensive, not least because of the complex safety precautions required.

It would therefore be desirable if butenes could be provided at only one site without transport over relatively large distances in amounts for the oligomerization as are required for the operation of a large scale plant for preparing nonanols, for example having a capacity of 200,000 to 800,000 metric t/year. It would further be desirable to have a process for preparing butene oligomers in which the valuable di-iso-butene can be separated off from the di-butene. The di-iso-butenes can be hydrogenated to yield di-s, which are valuable fuel additives as a substitute for methyl-tert.-butyl-ether. Finally, it would be desirable if the process could be controlled in such a manner that, in addition to higher butene oligomers, only di-iso-butene is formed as di-butene.

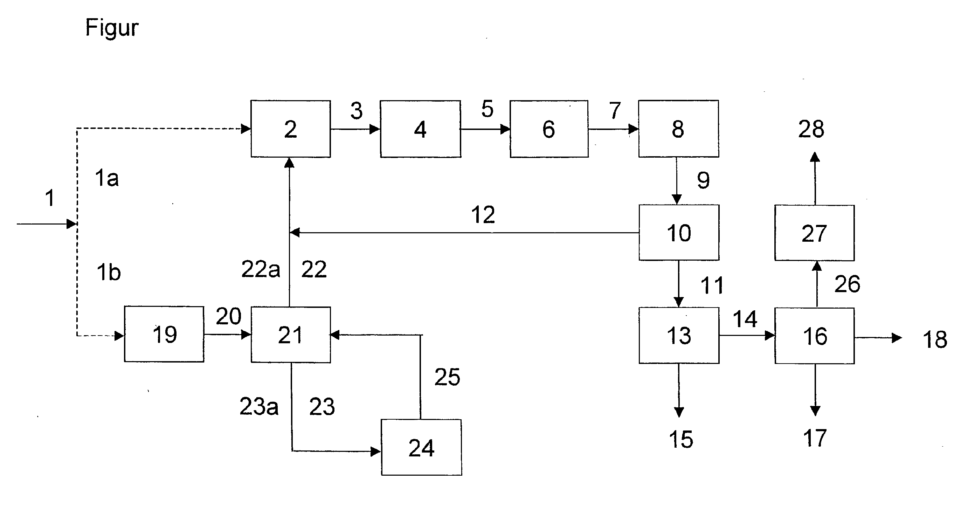

The process according to the invention is described in more detail by the block diagram of the accompanying figure, in which the Variants A and B described in more detail below are listed together with their obligatory and optional process stages. The field butane **1** is assigned as stream **1a** to the Variant A; the alternative stream **1b** belongs to the Variant B.

The invention is therefore a process for preparing oligomers and di-ios-butanes from field butanes which comprises
(a) dehydrogenating the n-butane and iso-butane present in the field butanes **1** in a dehydrogenation stage **2** and
(b) oligomerizing the dehydrogenation mixture **3** in an oligomerization stage **8** to give an oligomerization mixture **9** and
(c) separating di-iso-butene, di-n-butene and residual gas from the oligomerisation mixture and
(d) hydrogenation of di-iso-butenes to give di-iso-butanes.

This process is termed Variant A below.

In detail, the di-butene **14** is separated off from the oligomers **11,** which remain after separating off the residual gases **12** from the oligomerization mixture **9.** After separation of the di-butene **14,** di-n-butene **17**, and/or di-iso-butene **26** can be obtained separately by means of the destillation stage **16.**

In Variant **B,** the process can be controlled in such a manner that, in addition to higher butene oligomers, only di-iso-butene is formed, by separating off iso-butane **22a** by fractional distillation from the, if appropriate previously hydrogenated, field butane **1,** isomerizing the remaining n-butane **23a** in an isomerization stage **24** to give a mixture of n-butane and iso-butane separating off the iso-butane from the isomerization mixture **25** by fractional distillation and conducting it into the dehydrogenation stage **2**, together with the iso-butane **22a** separated off directly from the field butane **1** and recycling the remaining n-butane **23a** into the isomerization stage **24.**

The process according to the invention with its Variants A and B is distinguished by high flexibility. Therefore, depending on market requirements, if desired, only di-n-butene, di-butene, di-iso-butene, and other di-butenes conjointly or only di-isobutene in addition to di-iso-butane can be produced.

The term field butanes is applied to the C₄ fraction of the "moist" portions of the natural gas and of the crude oil-associated gases which are separated off from the gases in liquid form by cooling to about -30°C. Low-temperature distillation produces therefrom the field butanes, whose composition fluctuates with the field, but which generally contain about 30% iso-butane and 65% n-butane. Further components are generally about 2% C_{<4} hydrocarbons and about 3% C_{>4} hydrocarbons. Field butanes can be used without separation as feedstuffs in steam crackers or as an additive to motor gasoline. They may be resolved into n-butane and iso-butane by fractional distillation. Iso-butane is used, e.g., to an important extent for preparing propylene oxide by cooxidation of propylene and iso-butane and is also used as alkylating agent, by which n-butene or isobutene is alkylated to give isooctane which is valued as an additive to motor gasoline because of its high octane rating. In contrast, n-butane has only found less important uses. It serves, e.g., as butane gas for heating purposes or is used in relatively small amounts, e.g. for preparing polymers or copolymers or maleic anhydride by atmospheric oxidation. Formerly, n-butane was also dehydrogenated via the n-butene stage to give 1,3-butadiene, but this process has become uneconomic in the interim.

Because iso-butane is the more sought-after component of the field butane, n-butane is isomerized on a large scale to iso-butane (cf., e.g. R. A. Pogliano et. al., Dehydrogenation-based Ether Production, 1996 Petrochemical Review. DeWitt & Company, Houston, Texas, Butamer^{(R)} process, page 6; and S. T. Bakas, F. Nierlich et al., Production of Ethers from Field Butanes and Refinery Streams, AIChE Summer Meeting, 1990, San Diego, California, page 11).

### Variant A

The field butanes **1a** are first dehydrogenated in the dehydrogenation stage **2**. The dehydrogenation is a codehydrogenation. It is remarkable that the dehydrogenation of the field butane which is a mixture of components having different dehydrogenation behaviors succeeds so readily. The process conditions substantially correspond to those as are known for n-butane and iso-butane or other lower hydrocarbons. Thus, S. T. Bakas, F. Nierlich et al., loc. cit., pages 12 ff., describe the Oleflex^{(R)} process which is generally suitable for the selective preparation of light olefins and by which iso-butane can be dehydrogenated to isobutene with a selectivity of 91 to 93%. Further relevant publications are those of G. C. Sturtevant et al., Oleflex - Selective Production of Light Olefins, 1988 UOP Technology Conference and EP 0 149 698. The dehydrogenation is expediently carried out in the gas phase on fixed-bed or fluidized catalysts, e.g. on chromium (III) oxide or, advantageously, on platinum catalysts having aluminum oxide or zeolites as support. The dehydrogenation generally takes place at temperatures of 400 to 800°C, advantageously from 550 to 650°C. Atmospheric pressure or slightly elevated pressure of up to 3 bar is generally employed. The residence time in the catalyst bed is generally between 1 and 60 minutes, depending on catalyst, temperature and desired degree of conversion. The throughput is accordingly generally between 0.6 to 36 kg of field butane per m³ of catalyst and hour.

It is expedient to carry out the dehydrogenation only until about 50% of the n-butane and iso-butane remain unchanged in the dehydrogenation mixture **3**. Although higher degrees of conversion can be attained at higher temperature, cracking reactions which decrease the yield proceed to an increasing extent, owing to coke deposits, which reduce the service life of the dehydrogenation catalyst. The optimum combinations of the reaction conditions which lead to the desired degrees of conversion, such as type of catalyst, temperature and residence time, may be determined without difficulty by preliminary experiments.

The dehydrogenation mixture **3** generally contains 90 to 95% C₄ hydrocarbons and, in addition, hydrogen and lower- and higher-boiling portions which in part originate from the field butane **1**, and in part are formed in the dehydrogenation stage **2.** Purification is expediently performed upstream of the oligomerization. In a first purification stage (not shown in the figure), the C₄ fraction and the higher-boiling portions are condensed out. The condensate is distilled under pressure, co-condensed dissolved C_{<4} hydrocarbons passing overhead. From the bottom product, in a further distillation the C₄ hydrocarbons are obtained as main product and the comparatively small amount of C_{>4} hydrocarbons is obtained as residue.

The C₄ hydrocarbons, depending on the degree of conversion, generally contain small amounts, such as 0.01 to 5 % by volume, of 1,3-butadiene. It is advisable to remove this component since, even in markedly lower amounts, it can damage the oligomerization catalyst. A suitable process is selective hydrogenation **4** which, in addition, increases the proportion of the desired n-butene. A suitable process has been described, e.g., by F. Nierlich et. al. in Erdöl & Kohle, Erdgas, Petrochemie, **1986,** pages 73 ff. It operates in the liquid phase with completely dissolved hydrogen in stoichiometric amounts. Selective hydrogenation catalysts which are suitable are, e.g., nickel and, in particular, palladium, on a support, e.g. 0.3 per cent by weight of palladium on activated carbon or, preferably, on aluminum oxide. A small amount of carbon monoxide in the ppm range promotes the selectivity of the hydrogenation of 1,3-butadiene to give the monoolefin and counteracts the formation of polymers, the so-called "green oil", which inactivate the catalyst. The process generally operates at room temperature or elevated temperatures up to about 60°C and at elevated pressures which are expediently in the range of up to 20 bar. The content of 1,3-butadiene in the C₄ fraction of the dehydrogenation mixture is decreased in this manner to values of <1 ppm.

It is further expedient to pass the dehydrogenation mixture **5** C₄ fraction, which is then substantially freed from 1,3-butadiene, via the purification stage **6**, a molecular sieve, upstream of the oligomerization stage, as a result of which further substances which are harmful for the oligomerization catalyst are removed and its service life is further increased. These harmful substances include oxygen compounds and sulfur compounds. This process has been described by F. Nierlich et al. in EP-B1 0 395 857. A molecular sieve having a pore diameter of 4 to 15 angstroms, advantageously 7 to 13 angstroms, is expediently used. In some cases it is expedient for economic reasons to pass the dehydrogenation mixture successively over molecular sieves having different pore sizes. The process can be carried out in the gas phase, in liquid phase or in gas-liquid phase. The pressure is accordingly generally 1 to 200 bar. Room temperature or elevated temperatures up to 200°C are expediently employed.

The chemical nature of the molecular sieves is less important than their physical properties, i.e. in particular the pore size. The most diverse molecular sieves can therefore be used, both crystalline natural aluminum silicates, e.g. sheet lattice silicates, and synthetic molecular sieves, e.g. those having a zeolite structure. Zeolites of the A, X and Y type are available, inter alia, from Bayer AG, Dow Chemical Co., Union Carbide Corporation, Laporte Industries Ltd., and Mobil Oil Co. Suitable synthetic molecular sieves for the process are also those which, in addition to aluminum and silicon, also contain other atoms introduced by cation exchange, such as gallium, indium or lanthanum, as well as nickel, cobalt, copper, zink or silver. In addition, synthetic zeolites are suitable in which, in addition to aluminum and silicon, still other atoms, such as boron or phosphorus, have been incorporated into the lattice by mixed precipitation.

As already stated, the selective hydrogenation stage **4** and the purification stage **6** using a molecular sieve are optional, advantageous measures for the process according to the invention. Their order is in principle optional, but the order specified in the figure is preferred.

The dehydrogenation mixture **7,** if appropriate pretreated in the described manner, is passed into the oligomerization stage **8** which is an essential part of the process according to the invention. The oligomerization is a co-oligomerization of n-butenes and isobutene which is carried out in a manner known per se, such as has been described, e.g., by F. Nierlich in Oligomerization for Better Gasoline, Hydrocarbon Processing, **1992,** pages 45 ff, or by F. Nierlich et al. in the previously mentioned EP-B1 0 395 857. The procedure is generally carried out in the liquid phase and, as homogeneous catalyst, a system is employed, e.g., which comprises nickel (II) octoate, ethylaluminum chloride and a free fatty acid (DE-C 28 55 423), or preferably one of the numerous known fixed-bed catalysts or catalysts suspended in the oligomerization mixture which are based on nickel and silicon. The catalysts frequently additionally contain aluminum. Thus, DD-PS 160 037 describes the preparation of a nickel- and aluminum-containing precipitated catalyst on silicon dioxide as support material. Other useful catalysts are obtained by exchanging positively charged particles, such as protons or sodium ions, which are situated on the surface of the support materials, for nickel ions. This is successful with the most diverse support materials, such as amorphous aluminum silicate (R. Espinoza et al., Appl. Kat., **31** (1987) pages 259 - 266; crystalline aluminum silicate (DE-C 20 29 624; zeolites of the ZSM type (NL Patent 8 500 459; an X zeolite (DE-C 23 47 235); X and Y zeolites (A. Barth et al., Z. Anorg. Allg. Chem. **521**, (1985) pages 207 - 214); and a mordenite (EP-A 0 281 208).

The co-oligomerization is expediently carried out, depending on the catalyst, at 20 to 200°C and under pressures of 1 to 100 bar. The reaction time (or contact time) is generally 5 to 60 minutes. The process parameters, in particular the catalyst type, the temperature and the contact time, are matched to one another in such a manner that the desired degree of oligomerization is attained. In the case of nonanols as desired target product, this is predominantly a dimerization. For this purpose, clearly the reaction must not proceed to full conversion, but conversion rates of 30 to 70% per pass as expediently sought after. The optimum combinations of the process parameters may be determined without difficulties by preliminary experiments.

The residual gas **12** is separated off from the oligomerization mixture **9** in a separation stage **10** and recycled to the dehydrogenation stage **2.** If a catalyst of the liquid catalyst type mentioned was used in the oligomerization stage **8,** the residual gas **12** should be purified in advance to protect the dehydrogenation catalyst. The oligomerization mixture is initially treated with water, in order to extract the catalyst components. The residual gas **12** separated off is then dried with a suitable molecular sieve, other minor components also being separated off. Then polyunsaturated compounds, such as butynes, are removed by selective hydrogenation, e.g. on palladium catalysts, and the residual gas **12** thus purified is recycled into the dehydrogenation stage **2.** These measures for purifying the residual gas **12** are unnecessary if a solid oligomerization catalyst is used.

The oligomers **11** remaining after separating off the residual gas **12** are suitable, because of their branched components, as an additive to motor gasoline to improve the octane rating.

The oligomers **11** are separated in the distillation stage **13** into di-butenes **14** and trimers **15,** i.e. isomeric dodecenes, and yet higher oligomers, the main fraction comprising the desired di-butenes **14.** The dodecenes **15** can be hydroformylated, the hydroformylation products can be hydrogenated and the tridecanols thus obtained can be ethoxylated, as a result of which valuable detergent bases are obtained.

The di-butenes **14** are separated in the fine distillation stage **16** into di-n-butene **17,** di-iso-butenes **26,** and the residual di-butenes **18** which, as more highly branched molecules are lower boiling, which residual di-butenes can likewise be used for preparing nonanols or can be added prior or after hydrogenation to motor gasoline. This procedure is a more expedient alternative to the variant in which n-butene and isobutene are separated off from the co-dehydrogenation mixture **7** by distillation and these isomers are oligomerized separately. This variant would require two separate oligomerization stages, which would be considerably more capital-intensive and also more complex in operation than only one, all be it larger, co-oligomerization stage **8** in combination with a fine distillation stage **16.**

### Variant B

This variant is selected when it is desired to prepare only di-iso-butene as di-butene. If the field butane **1b** contains olefinically unsaturated components, it is advantageously first passed into a hydrogenation stage **19,** because these components can interfere with the later isomerization of the iso-butane. The hydrogenation proceeds in a manner known per se, such as described by K. H. Walter et al., in the Hüls Process for Selective Hydrogenation of Butadiene in Crude C₄'s, Development and Technical Application, DGKM meeting Kassel, November 1993. The procedure is therefore expediently carried out in the liquid phase and, depending on the catalyst, at room temperature or elevated temperature up to 90°C and at a pressure of 4 to 20 bar, the partial pressure of the hydrogen being 1 to 15 bar. The catalysts customary for the hydrogenation of olefins are used, e.g. 0.3% palladium on aluminum oxide.

The hydrogenated field butanes **20** are passed into the separation stage **21.** This generally comprises a highly effective column in which n-butane **22** and iso-butane **23** are separated by fractional distillation. The column **21** is operated in a customary manner, expediently at a pressure of from 4 to 7 bar. The isomerization of n-butane and iso-butane is a known reaction (see, e.g., H. W. Grote, Oil and Gas Journal, **56** (13 pages 73 ff., (1958)). The procedure is generally carried out in the gas phase, expediently at a temperature of 150 to 230°C at a pressure of 14 to 30 bar and using a platinum catalyst on aluminum oxide as support, whose selectivity can be further improved by doping with a chlorine compound, such as carbon tetrachloride. Advantageously, a small amount of hydrogen is added, in order to counteract a dehydrogenation. The selectivity of the isomerization to iso-butane is high; cracking to form smaller fragments only takes place to a minor extent (approximately 2%).

The isomerization mixture **25** must be separated into the isomers. This is expediently performed in the column **21** which is present in any case, from which passes into the dehydrogenation stage **2** which, in contrast to Variant A, is not a co-dehydrogenation stage. The **22a** is passed from the column **21** into the dehydrogenation stage **2,** in which no co-dehydrogenation takes place. The n-butane **23a** is passed from the column **21** into the isomerization stage **24** and there isomerized to at most up to equilibrium. The iso-butane is separated from n-butane, expediently in the column **21,** and passed into the dehydrogenation stage **2,** whereas the n-butane returns to the isomerization stage **24.** In this manner, the n-butane is completely converted into iso-butane. The dehydrogenation mixture **3** is expediently purified as described in Variant A. The oligomerization in the oligomerization stage **8** is a homo-oligomerization, because only iso-butene participates therein, and di-isobutene arises in the distillation stage **13.** The fine distillation **16** is likewise omitted.

In both variants, the resulting di-iso-butenes **26** can be hydrogenated in stage **27** to give di-iso-butane **28.** Both compounds, **26** and **28,** can be used as fuel additives to increase octane rating. The hydrogenation stage **27** can be performed as known in the art e.g. via a nickel-containing catalyst or in the same manner as the hydrogenation stage **19.**

## Claims

1. A process for preparing butene oligomers and di-iso-butane from field butanes, which comprises
(a) dehydrogenating the n-butane and iso-butane present in the field butanes **1** in a dehydrogenation stage **2** and
(b) oligomerizing the dehydrogenation mixture **3** in an oligomerization stage **8** and
(c) separating di-iso-butene, di-n-butene and residual gas from the oligomerisation mixture and
(d) hydrogenation of di-iso-butenes to give di-iso-butanes.

2. The process as claimed in claim 1, wherein a selective hydrogenation **4** and/or a purification stage **6** using a molecular sieve is arranged in any order between the dehydrogenation stage **2** and the oligomerization stage **8.**

3. The process as claimed in either of claims 1 or 2, wherein the residual gas **12** is separated off from the oligomerization mixture **9** and, if appropriate after purification, is recycled into the dehydrogenation stage **2.**

4. The process as claimed in claim 3, wherein the di-butene **14** is separated off from the oligomers **11** remaining after separating off the residual gas **12.**

5. The process as claimed in claim 4, wherein the di-butenes **14** are separated in a fine distillation stage **16** into di-n-butenes **17,** di-iso-butene **26,** and residual di-butenes **18.**

6. The process as claimed in one of claims 1 to 5, wherein only di-iso-butene **26** is produced, by separating off iso-butane from the, if appropriate prehydrogenated, field butane **1** by fractional distillation and passing it into the dehydrogenation stage **2,** isomerizing the remaining n-butane in an isomerization stage **24** to give a mixture of n-butane and iso-butane, separating off the iso-butane from the isomerization mixture **25** by fractional distillation and conducting it into the dehydrogenation stage **2** together with the iso-butane separated off directly from the field butane, and recycling the remaining n-butane into the isomerization stage **24.**

7. The use of the di-n-butenes **16,** the residual di-butenes **17** as claimed in claims 1 to 5 for preparing nonanols by hydroformylation and subsequent hydrogenation.

8. The use of the di-n-butenes **16** as claimed in claims 1 to 5 for preparing nonanoic acids by hydroformylation and subsequent oxidation.

9. The use of the di-iso-butanes as claimed in claims 1 to 6 as fuel additive.
